# EUROPÄISCHE PATENTSCHRIFT

(11) **EP 2 323 572 B1**
(45) Veröffentlichungstag und Bekanntmachung des Hinweises auf die Patenterteilung: **02.11.2016**
(21) Anmeldenummer: 09765315.8
(22) Anmeldetag: 17.06.2009
(51) Int. Cl.: A61B 17/64

(54) **VORRICHTUNG ZUR EXTERNEN FIXIERUNG VON KNOCHENBRÜCHEN**
DEVICE FOR EXTERNALLY FIXING BONE FRACTURES
DISPOSITIF DE FIXATION EXTERNE DE FRAGMENTS D'OS

(30) Priorität: 17.06.2008 CH 929082008
(43) Veröffentlichungstag der Anmeldung: 25.05.2011
(73) Patentinhaber: Durst, Heiko, 93138 Lappersdorf (DE)
(72) Erfinder: LORENZ, Kai-Uwe, 8245 Feuerthalen/ZH (CH); DURST, Heiko, 9042 Speicher (CH)
(74) Vertreter: Glück Kritzenberger Patentanwälte PartGmbB
(86) Internationale Anmeldenummer: PCT/CH2009/000208
(87) Internationale Veröffentlichungsnummer: WO 2009/152633

(56) Entgegenhaltungen:
- EP-A- 0 807 419
- WO-A-99/20194
- JP-A- 2004 298 253
- US-A1- 2003 187 432
- US-A1- 2009 018 541

## Beschreibung

### Technisches Gebiet

Die Erfindung betrifft eine Vorrichtung zur externen Fixierung von Knochenbrüchen, gemäß dem Oberbegriff des unabhängigen Patentanspruchs 1.

### Stand der Technik

Ein Fixateur externe ist eine ausserhalb des Körpers angebrachte Vorrichtung, die über Metall-Gewindestangen, so genannte Pins, am Knochen Fixiert ist, zur externen Fixierung von Knochenbrüchen eines Patienten, und wird in der Regel bei offenen Frakturen mit Weichteilschädigungen oder bei Mehrfachverletzten angewendet. Bei Notfalloperationen von Mehrfachverletzten ist es oft notwendig, die definitive Versorgung von Knochenbrüchen an Extremitäten, beispielsweise an Oberschenkel oder Unterschenkel, zugunsten lebensbedrohlicher, weiterer Verletzungen zu verschieben, um das Leben des Patienten nicht zu riskieren. Nach einer ersten Reposition der Fraktur wird diese mit einem äusseren Gestell, dem Fixateur externe, provisorisch stabilisiert. Bei offenen Brüchen wiederum kann es ebenfalls notwendig sein, die Fraktur mit einem Fixateur externe zu stabilisieren, da eine definitive primäre Bruchversorgung mittels Platte, Schraube oder Nagel die Gefahr einer postoperativen Infektion erhöhen würde. Der spätere Abbau des Fixateur externe und die definitive Knochenstabilisierung mittels Platte und Schrauben oder mit Nägeln erfolgt bei offenen Brüchen nach Erreichen einer sauberen Wunde, bzw. bei mehrfachverletzten Patienten nach Erreichen eines stabilen Zustands.

Beim so genannten Pin-Fixateur werden Pins, zum Beispiel Schanz'sche Schrauben oder Steinmann-Nägel im Knochengewebe verankert. Solche Pins sind in der Regel Stahlstifte mit standardisierten Abmessungen. Nach erfolgtem Einrichten des Knochenbruches werden die Pins mit einem starren äusseren Gestell fest miteinander verbunden, dass häufig aus miteinander verbundenen Stangen oder Röhren besteht. Alternative Formen sind der Zangen-Fixateur, der Ring- bzw. Halo-Fixateur, und kombinierte Vorrichtungen, so genannte Hybridfixateure.

Wenn ein Patient in die Notfallaufnahme eingeliefert wird, der eine höhergradige offene Fraktur (Grad 3°b oder 3°c) erlitten hat, darf diese Fraktur nicht direkt mit Platte oder Nagel stabilisiert werden. Stattdessen wird in diesen Fällen vorerst ein Fixateur externe montiert, nach einigen Tagen erfolgt dann der Verfahrenswechsel. Der Fixateur externe wird abgebaut und eine definitive Osteosynthese zum Beispiel mittels Platte oder Nagel erfolgt. Die Montage eines Fixateur externe ist bei den bekannten Modellen oft sehr zeitaufwendig, da zur Montage nach der Einrichtung des Bruches in der Regel viele Schrauben angezogen und bei ungenügender Stellung zur Korrektur wieder gelöst und neu angezogen werden müssen. Wenn mehrere Stellungskorrekturen notwendig sind, kann dieser Vorgang sehr lange dauern. Da es sich in Notfallsituationen bei Patienten mit höhergradig offenen Frakturen oft um Schwerverletzte handelt, mit Zusatzverletzungen beispielsweise an Brustkorb oder Bauch, sollte die erste Versorgung des Knochenbruchs so schnell als möglich abgeschlossen werden, damit der Patient möglichst bald auf die Intensivstation verlegt werden kann. Die bisherigen Fixateur-Systeme lassen dies aus den oben genannten Gründen nicht zu. Verschiedene Ausführungsformen von Fixateurs externes erschweren zudem die anatomische Einstellung der Bruchfragmente, da vier oder mehr Knochenpins auf einer Geraden ausgerichtet werden müssen, was aufgrund der Pin-Position erschwert oder verunmöglicht sein kann. Zudem ist die Bauweise der bekannten Stangen- und Röhren-Fixateur-Systeme oft voluminös und sperrig.

Es wurde eine Reihe von Fixateur-Systemen entwickelt, die eine bessere Anpassung des Fixateur externe an die jeweiligen speziellen Gegebenheiten eines Bruchs erlauben sollen.

DE 10125742 C1 zeigt einen Fixateur externe, bei dem eine Reihe von verschiedenen, mit einer Durchgangsöffnung versehenen Einzelmodulen auf ein Spannseil aufgezogen werden. Die verschiedenen Typen von Einzelmodulen weisen unterschiedliche Längen und/oder Anschlusswinkel auf. Zudem sind spezielle Anschlusselemente für Knochen-Pins und Haltestangen vorgesehen. Die einzelnen Elemente verfügen an einer stirnseitigen Anschlussfläche jeweils über vier Zapfen und an der gegenüberliegenden Seite über vier Vertiefungen, so dass jeweils zwei aufeinander folgende Einzelmodule mit vier verschiedenen Drehwinkeln zueinander formschlüssig angeordnet werden können. Nach dem Anpassen des Fixateurs wird das Spannseil verspannt. Die Einzelmodule sind nun auch in Richtung des Spannseils kraftschlüssig miteinander verbunden. Der genannte Fixateur externe erlaubt nur eine relative grobe Anpassung an die anatomischen Verhältnisse. Nach dem Auffädeln auf das Spannseils ist zudem eine Feinjustierung bzw. Anpassung an die Lage der Pins nicht mehr möglich. So ist den auch vorgesehen, dass die Knochenschrauben erst nach dem Erstellen des Fixateurs in den Knochen eingedreht werden.

US 5944719 zeigt einen Fixateur externe, bei dem eine Kette von Kugelgelenken und Manschetten mit einem Spannseil gespannt und unter Zug versteift wird. Eine Mehrzahl von Manschettenelementen verfügt über schwenkbare Pin-Haltevorrichtungen, mit welchem im Knochengewebe angebrachte Pins am Fixateur externe befestigt werden können. Dazu muss der Pin mit seinem distalen Ende über eine Mutter mit den Pin-Haltevorrichtungen verbunden werden. Der Abstand des Fixateurs vom Knochen ist also von der Länge der Pins vorgegeben, die darum nur kurz sein dürfen. Aufgrund der Kugelgelenke ist der Fixateur beweglich in der Formgebung genau an die anatomische Situation anpassbar. Da jedoch die Position der Haltevorrichtungen auf dem Fixateur vorgegeben ist, bzw. der Fixateur beim Zusammenbau entsprechend vorbereitet werden muss, ist die genannte Vorrichtung vor allem für vorbereitete Operationen geeignet, jedoch weniger für eine Erstversorgung unter hohem Zeitdruck.

Eine ähnliche Vorrichtung mit einer durch mittels eines Spannseils versteifbaren Kette von Kugelgelenken zeigt WO 99/20194 A1. In einer Ausführungsform werden anstelle der mit den Pin-Haltevorrichtungen ausgestatteten Kugelgelenkelemente nachträglich an der Fixateurkette anbringbare Manschetten mit Pin-Haltevorrichtungen verwendet, so dass die Lage der Haltevorrichtungen schneller an die tatsächliche Lage der Pins angepasst werden kann.

WO 03/068085 A1 zeigt eine Variante eines Fixateurs mit Kugelgelenkelementen analog zur oben genannten Vorrichtung, bei welchem die Verbindungen zwischen den einzelnen Kugelgelenken mittels eines Schnellspannhebelverschlusses fixiert bzw. gelöst werden. Dies erlaubt einen sequentiellen Aufbau des Fixateurs aus einzelnen Teilen vor Ort. Jedoch kann bei dieser Ausführungsform die Gesamtform nicht so schnell angepasst werden, weil zuerst eine Vielzahl von Verschlüssen gelöst werden muss.

Aus der US 2003/187432 A1 ist eine Vorrichtung zur externen Fixierung von Knochenfragmenten bekannt. Die Vorrichtung umfasst eine Mehrzahl von modularen Komponenten, die jeweils aus einem nichtmetallischen Material gebildet sind. Dabei ist an einem Ende einer auf eine gewünschte Länge zugeschnittenen Stange ein Verriegelungselement angebracht, welches zum Eingriff in eine weitere Stange vorgesehen ist. Durch Kombination von Klemmen, Stiften, Abstandhaltern und Federn wird die gewünschte Fixerung der Knochenfragmente erreicht.

Die JP 2004 298253 A beschreibt eine externe Knochen-Fixierungsvorrichtung mit einer Mehrzahl von Stiften, die in einem Knochen gesteckt werden, einer Vielzahl von Kugelgelenkmitteln, die gegeneinander und gegen die Stifte verdrehbar ausgebildet sind, und Befestigungsschrauben zur Fixierung der Stifte an den Kugelgelenkmitteln. Durch Lösen der Befestigungsschrauben können die Stifte im jeweils gewünschten Winkel eingestellt werden.

Aus der EP 807 419 A2 ist ein externer Fixateur bekannt, der insbesondere zur Fixierung von Brüchen kleiner Knochen bei Kindern geeignet ist. Der Fixateur umfasst ein Paar Klemmen für Knochenbolzen, die mittels sicherbarer Kugelgelenke mit einem zentralen Körper einstellbarer Länge verbunden sind, der mindestens ein männliches Element und ein weibliches Element umfasst. Eines der teleskopisch ausgebildeten weiblichen Elemente ist aus einem Material hergestellt, das in hohem Maße für Röntgenstrahlen durchlässig ist, während ein anderes der teleskopischen Elemente aus einem im wesentlichen strahlungsundurchlässigen starren Material hergestellt ist. Die Seitenwände dieses teleskopischen Elements weisen eine so geringe Dicke auf, dass sie zumindest teilweise durchlässig für Röntgenstrahlen sind, um so eine Radioskopie des Bruchs durch den zentralen Körper hindurch zuzulassen.

### Darstellung der Erfindung

Aufgabe der Erfindung ist es, eine Vorrichtung der eingangs erwähnten Art zur Verfügung zu stellen, welche die oben erwähnten und andere Nachteile nicht aufweist. Insbesondere soll eine solche Vorrichtung zur externen Fixierung von Knochenbrüchen ein einfaches Einrichten eines Knochenbruches mit in den Knochen, respektive die Knochenfragmente, eingebrachten Pins und anschliessend ein schnelles, formstabiles Versteifen der Vorrichtung erlauben. Eine erfindungsgemässe Vorrichtung soll bevorzugt einfach und mit wenigen Handgriffen zu bedienen sein, auch unter Stressbedingungen. Bei ungenügender Reposition soll die erfindungsgemässe Vorrichtung ebenso leicht wieder entriegelbar sein, um eine Korrektur der Reposition und ein anschliessendes erneutes Versteifen zu erlauben. Eine erfindungsge-mässe Vorrichtung soll bevorzugt auch grösstenteils röntgendurchlässig sein, um eine Kontrolle der Reposition zu erlauben. Zudem sollte sie bevorzugt auch in einem Kernspintomografen eingesetzt werden zu können.

Diese und andere Aufgaben werden gelöst durch eine erfindungsgemässe Vorrichtung zur externen Fixierung von Knochenbrüchen, gemäß dem Oberbegriff des unabhängigen Patentanspruchs 1. Weitere bevorzugte Ausführungsformen sind in den abhängigen Ansprüchen gegeben.

Eine erfindungsgemässe Vorrichtung zum Fixieren von Knochenbrüchen besteht aus einer versteifbaren Stütze aus einzelnen Gelenkelementen, welche auf einem zentralen, flexiblen Zugkraftelement aufgereiht sind. Jeweils zwei benachbarte Gelenkelemente bilden dabei ein Kugelgelenk. Herkömmliche Pins, welche vorab in den einzelnen zu fixierenden Knochenfragmenten verankert sind, werden durch die Gelenkelemente klemmend gehalten, und so die Knochenfragmente zueinander räumlich stabilisiert. Das Festklemmen der Pins und das Versteifen der Vorrichtung erfolgt gleichzeitig, durch das Beaufschlagen des zentralen Zugkraftelements mit einer ausreichend hohen Zugkraft. Dabei werden die Auflageflächen der Kugelgelenke aufeinander gedrückt, wodurch eine stabile reibschlüssige Verbindung zwischen den einzelnen Gelenkelementen der Vorrichtung entsteht, und die Vorrichtung vollständig versteift wird. Gleichzeitig werden die Pins festgeklemmt, entweder zwischen zwei benachbarten Gelenkelementen, oder mit zweiteiligen Gelenkelementen. Durch das Lösen der Zugkraft wird die Vorrichtung wieder entriegelt und die Verbindung zu den Pins gelöst.

In einer bevorzugten Variante einer erfindungsgemässen Vorrichtung ist im entriegelten Zustand das Zugkraftelement bereits mit einer gewissen Zugkraft vorgespannt, so dass bei der Montage der Vorrichtung diese an den Pins nach dem Einklinken provisorisch hält, und trotzdem beim Einrichten des Bruchs die Fixateur-Vorrichtung vom Operateur leicht in die gewünschte Form gebracht werden kann. Nach dem Loslassen hält die erfindungsgemässe Vorrichtung provisorisch die Form.

Sowohl für das Zugkraftelement als auch für die Celenkelemente können sämtliche geeigneten Werkstoffe, insbesondere Metalle und faserverstärkte Kunststoffe, verwendet werden. Für die Zugkraftelemente werden jedoch bevorzugt Drähte, Bänder, Seile oder besonders bevorzugt Faserbündel (im folgenden auch kurz Bündel genannt) aus Metall, Kohlefaser oder aus anderen geeigneten Kunststoffen oder Kombinationen der vorgenannten verwendet, welche bei geringem Gewicht sehr hohe Zugkräfte aufnehmen können, ohne sich auch über einen Zeitraum von Monaten zu verformen. Für die Kugelgelenkelemente werden bevorzugt faserverstärkte Polymerwerkstoffe verwendet, um eine hohe mechanische Stabilität bei gleichzeitig geringem Gewicht sicherzustellen. Eine solche Wahl der Werkstoffe hat zudem den Vorteil, dass die erfindungsgemässe Vorrichtung röntgendurchlässig ist, und auch keine metallischen Teile aufweist, welche in einem Kernspintomografen Artefakte verursachen oder deren Anwendung im Magnetfeld des Kernspintomografen verbieten würden.

Bei einem Verfahren zum Fixieren von Knochenbrüchen werden herkömmliche Pins im Knochengewebe des Patienten verankert. Normalerweise werden zwei Pins je grosses Knochenfragment verwendet, also insgesamt mindestens vier Pins. Aus mechanischen Überlegungen wird der Abstand der Pins am gleichen Fragment möglichst gross gewählt werden. Anschliessend werden die Pins an einer erfindungsgemässen Fixateur-Vorrichtung eingeklinkt. Die einzelnen Elemente der Vorrichtung sind im entriegelten Zustand gegeneinander schwenkbar beweglich und folgen den Bewegungen der Pins bei der Reposition des Knochens durch den Operateur. Durch das Spannen eines zentralen Zugkraftelements werden die Gelenkelemente gegeneinander verblockt und verbleiben auch unter Last in der vorher eingestellten Position. Dadurch wird der gebrochene Knochen in der eingestellten, möglichst ursprünglich-anatomischen Lage fixiert gehalten. Gleichzeitig werden die Pins in der gewünschten Lage positionsstabil an der Vorrichtung festgeklemmt.

Grundsätzlich ist die erfindungsgemässe Fixateur-Vorrichtung zur temporären Versorgung bis zur definitiven Fraktur-Versorgung mit Nägeln oder Platten vorgesehen, aufgrund ihrer Vorteile bezüglich Handling und Geschwindigkeit, vor allem in Notfallsituationen. Ein weiteres prädestiniertes Anwendungsgebiet sind Kriseneinsätze, wie beispielsweise Erdbeben, oder Kriegssituationen, bei denen schwer verletzte Patienten mit notdürftigen Mitteln in kürzester Zeit transportfähig gemacht werden müssen. Eine erfindungsgemässe Fixateur-Vorrichtung ist jedoch auch genauso gut für eine definitive Ausbehandlung einer Fraktur geeignet.

### Kurze Beschreibung der Zeichnungen

Im Folgenden wird die erfindungsgemässe Vorrichtung anhand von Zeichnungen erläutert.
- Figur 1: zeigt beispielhaft das hintere Ende einer erfindungsgemässen Vorrichtung, (a) in Seitenansicht, und (b) in einem Längsschnitt.
- Figur 2: zeigt einen Längsschnitt durch einen Abschnitt einer erfindungsgemässen Vorrichtung analog zu Figur 1, mit einer anderen Ausführungsform der Gelenkelemente.
- Figur 3: zeigt einen Längsschnitt durch einen Abschnitt einer erfindungsgemässen Vorrichtung analog zu Figur 2 bei maximalem Biegungswinkel.
- Figur 4: zeigt schematisch (a) einen mit einer erfindungsgemässen Vorrichtung stabilisierten Knochenbruch, und (b) eine Ausführungsform mit zwei Stützen und einem dazwischen angeordneten Zwischenelement.
- Figur 5: zeigt eine mögliche Ausführungsform eines einzelnen Gelenkelements einer erfindungsgemässen Vorrichtung, analog zu Figur 1.
- Figur 6: zeigt eine weitere mögliche Ausführungsform eines einzelnen Gelenkelements einer erfindungsgemässen Vorrichtung, analog zu Figur 2 und 3.
- Figur 7: zeigt eine zweiteilige Ausführungsform eines Gelenkelements für eine erfindungsgemässe Vorrichtung.
- Figur 8: zeigt eine weitere Variante einer erfindungsgemässen Vorrichtung mit zweiteiligem Gelenkelement.

### Ausführung der Erfindung

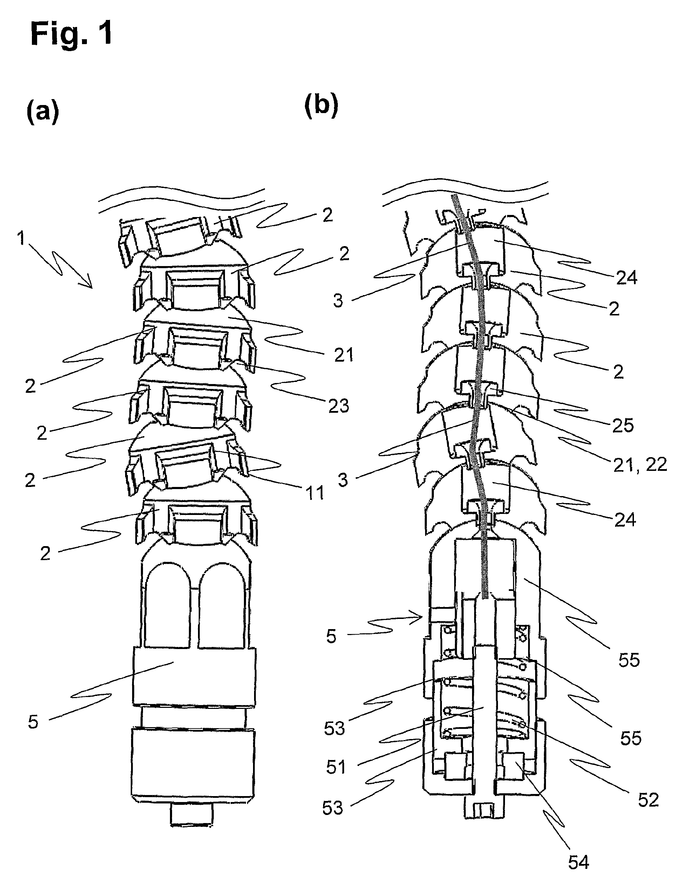

Eine mögliche Ausführungsform einer erfindungsgemässen Vorrichtung 1 zur externen Fixierung von Knochenbrüchen ist in Figur 1 dargestellt, (a) in Seitenansicht, und (b) in einem Längsschnitt. Gezeigt ist das hintere Ende der Stütze 11 der Vorrichtung 1, mit den sechs hintersten Gelenkelementen 2, und einer endständigen Spannungsvorrichtung 5 zur Erzeugung der zur Versteifung der Vorrichtung 1 notwendigen Zugkraft. Das über die gesamte Länge der Stütze 11 laufende Zugkraftelement 3 verläuft vom Spannungselement 5 durch die Längsdurchgänge 24 der Gelenkelemente 2, und endet an einem nicht dargestellten vorderen Ende der Stütze in einem Widerlager, welches eine anliegende Zugkraft aufnimmt, und auf die Gelenkelemente 2 überträgt. Das Zugkraftelement ist vorzugsweise als Faserbündel ausgestaltet, das bei möglichst geringer Dicke über eine möglichst hohe Zugfestigkeit verfügt. Geeignet sind dazu beispielsweise Drahtbündel, Kohlefaserbündel oder Bündel aus anderen geeigneten Kunststoffen. Letztere haben den zusätzlichen Vorteil der Röntgentransparenz und der Unempfindlichkeit gegenüber Magnetfeldern.

Die einzelnen Gelenkelemente 2 verfügen über eine obere Kugelgelenkfläche in Form einer Kugelkalotte 21 und eine untere Kugelgelenkfläche in Form einer Gelenkpfanne 22, wobei die Kugelkalotte 21 eines Gelenkelements 2 jeweils in der Gelenkpfanne 22 des nächsten Gelenkelements 2 aufliegt, so dass eine Abfolge von Kugelgelenken entsteht. Während der Montage der Fixateur-Vorrichtung 1 liegt keine oder nur eine reduzierte Zugkraft auf dem Zugkraftelement 3 an, so dass der Reibschluss zwischen den Kugelgelenkflächen 21, 22 zweier benachbarter Gelenkelemente 2 gering ist, und die einzelnen Kugelgelenke vom Operateur wie gewünscht ausgerichtet und angeordnet werden können. Bei einer Erhöhung der Zugkraft wird die Haftreibung zwischen den Kugelgelenkflächen 21, 22 so hoch, dass eine starke reibschlüssige Verbindung zwischen den einzelnen Gelenkelementen 2 entsteht, und die Vorrichtung 1 so versteift wird, dass sie nicht mehr zerstörungsfrei verformt werden kann.

Die Gelenkelemente können aus Metall oder einem geeigneten hochfesten Kunststoff gefertigt werden. Da sie an den Kontaktflächen 21, 22 über längere Zeit höheren Druckkräften ausgesetzt sind, sollte das Material über eine hohe Kriechfestigkeit verfügen. Speziell geeignet sind beispielsweise faserverstärkte Kunststoffe. Die Oberflächen, welche reibschlüssig aufeinander haften sollen, können geeignet behandelt werden, um den Reibungskoeffizienten zu erhöhen. So können diese beispielsweise aufgeraut oder beschichtet werden. Die Auflageflächen können auch mit einer Zahnung versehen werden.

Es ist möglich, die Orientierung der Kugelgelenkelemente umgekehrt zu wählen, so dass die Kugelkalotten gegen die Spannungsvorrichtung gerichtet sind. Ebenfalls ist auch eine Gestaltungsform möglich, bei der zwei verschiedene Typen Gelenkelemente abwechselnd aneinander gereiht sind, wobei ein erster Typ zwei Kugelkalotten aufweist, und ein zweiter Typ zwei Gelenkflächen aufweist.

Die Spannungsvorrichtung 5 dient dem Anspannen des Zugkraftelements 3. Eine Spannungsvorrichtung 5 kann verschieden realisiert werden, wobei vorzugsweise zum Erreichen der relativ hohen Zugkraft beim Versteifen nur ein geringer Kraftaufwand notwendig ist, und keine grösseren Hebelkräfte aufgewendet werden müssen, welche auf die Fraktur übertragen werden könnten. Im gezeigten Beispiel wird ein Gewindebolzen 51 mittels eines geeigneten Drehwerkzeugs wie z.B. Sechskant-Schlüssel, Akkuschrauber, Handkurbel, Drehmomentschlüssel gedreht, wodurch über eine Platte 54 mit einem Gegengewinde eine in einem Gehäuse 53 angeordnete Druckfeder 52 komprimiert wird. Um die Scherkraft des Drehwerkzeugs aufzufangen, muss die Spannungsvorrichtung 5, insbesondere das direkt mit dem ersten Gelenkelement 2 in Kontakt stehende Widerlager 55 fixiert werden, beispielsweise von Hand oder mit einer Zange. Die gegenwirkende Federkraft des Federelements 52 führt zu einer Kraftbeaufschlagung des Bolzens 51. Der Bolzen 51 ist über eine frei drehende Kopplung mit dem Zugkraftelement 3 verbunden, und überträgt damit die Federkraft auf das Zugkraftelement, und damit auf die Gelenkelemente 2. Dabei bewegen sich jedoch die einzelnen Gelenkelemente 2 nicht, so dass die Position der Pins bei der definitiven Stabilisation nicht verschoben wird. Wie im Folgenden noch ausführlicher beschrieben wird, spannt die Feder 52 unter Kraftbeaufschlagung die Gelenkelemente gegeneinander vor. Bei weiterem Anziehen des Gewindebolzens 51 wird das Federelement vollständig komprimiert und das Gehäuse 53 kommt mit einer Stirnfläche 53 an einer gegenüberliegenden Stufe 55 des Widerlagers 55 zu liegen. Der Federweg ist nun gleich Null und beim weiteren Anspannen des zentralen Zugkraft aufnehmenden Elements 3 werden die jeweils benachbarten Gelenkelemente entsprechend zunehmend stärker gegeneinander gepresst.

Im gezeigten Beispiel ist die Spannungsvorrichtung 5 ein integraler Bestandteil der erfindungsgemässen Vorrichtung 1. Alternativ ist es jedoch auch möglich, die Spannungsvorrichtung 5 zweiteilig zu gestalten, mit einem am hinteren Ende der Vorrichtung angeordneten Widerlager, welches der Aufrechterhaltung der Zugkraft dient, und einer Aktuatorvorrichtung, welche zum Erzeugen der Zugkraft dient, und zu diesem Zweck vorübergehend an der erfindungsgemässen Vorrichtung montiert wird. Aus dem Maschinenbau ist eine Vielzahl von Vorrichtungen zur Erzeugung einer Zugkraft bekannt. Fest montierte, einteilige Spannungsvorrichtungen sollten jedoch Platz sparend und leicht ausgestaltet sein, um nicht aufgrund ihres Volumens oder Gewichts zu stören. Dies schränkt die technischen Gestaltungs-Möglichkeiten ein, wobei aber dafür eine Montage der Vorrichtung ohne aufwendige Hilfsmittel möglich ist. Bei zweiteiligen Ausgestaltungen der Spannungsvorrichtung 5 ist dagegen die Erzeugung höherer Zugkräfte möglich, ohne das beim Erzeugen dieser Zugkräfte ein menschlicher Operateur einen grossen Krafteinsatz leisten muss, oder die erfindungsgemässe Vorrichtung beim Bedienen grossen Scherkräften ausgesetzt ist.

Alternativ kann beispielsweise ein Federelement mit einer geeigneten pneumatischen, hydraulischen oder elektrischen Aktuatorvorrichtung gespannt und anschliessend mit dem Zugkraftelement verriegelt werden, zum Beispiel mit einer Kontermutter, so dass anschliessend die Aktuatorvorrichtung wieder entfernt werden kann. Eine Aktuatorvorrichtung kann im einfachsten Fall beispielsweise auch ein pneumatisch oder elektrisch betriebener Drehschlüssel sein.

Bei einer weiteren möglichen Ausführungsform kann ein Federkraftelement auch bei einem am vorderen Ende der Vorrichtung angeordneten Widerlager angebracht werden. Anstatt separaten Federelementen kann auch die elastische Dehnung eines Stahlbolzens oder des Zugkraftelements selber als Federkraftelement verwendet werden. So kann beispielsweise ein mit dem Zugkraftelement verbundener Gewindebolzen mit einer definierten Zugkraft hydraulisch vorgespannt, danach der Bolzen mit einer Kontermutter ohne Kraftaufwand fixiert, und anschliessend die hydraulische Zugkraft wieder abgelassen werden. Ebenfalls möglich ist eine Spannungsvorrichtung, bei der nach erfolgter Montage der Vorrichtung ein Auslöser betätigt wird, der dazu führt, dass das Zugkraftelement und das Federelement augenblicklich mit der Zugkraft beaufschlagt, und damit die erfindungsgemässe Vorrichtung sofort versteift wird.

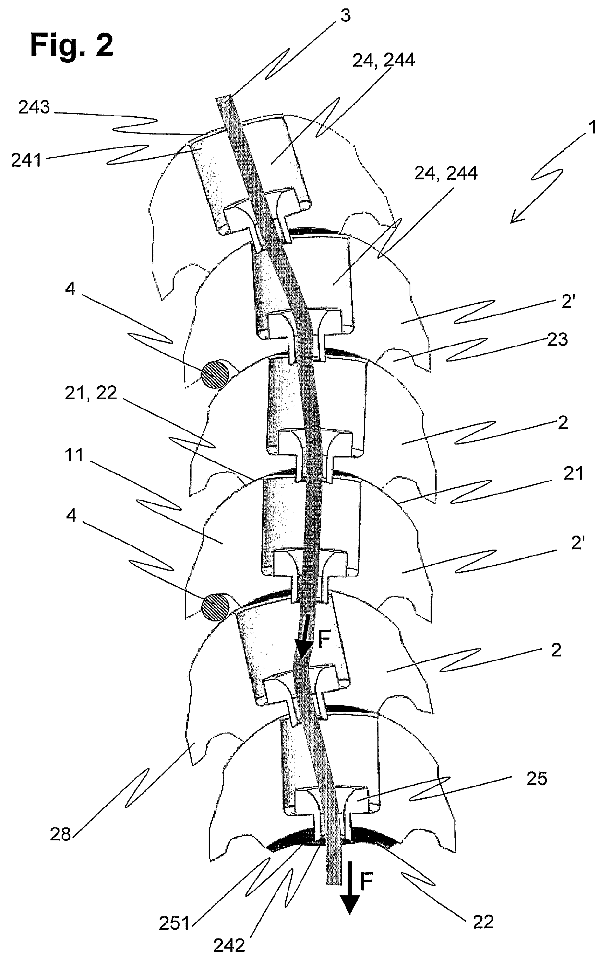

Figur 2 zeigt ebenfalls in einem Längsschnitt einen Abschnitt einer erfindungsgemässen Fixateur-Vorrichtung 1 analog zu Figur 1, mit zwei im Querschnitt dargestellten Pins 4, die jeweils zwischen zwei Gelenkelementen klemmend gehalten sind. Die zwei im Knochenwebe verankerten Pins 4 sind mit der Stütze 11 der Vorrichtung 1 kraft- und formschlüssig verbunden, indem sie zwischen der Kugelkalotte 21 eines ersten Gelenkelements 2 und einer Aufnahmenut 23 eines daran anschliessenden zweiten Gelenkelements 2' klemmend fixiert sind. Zur Montage der Pins 4 an der erfindungsgemässen Vorrichtung 1 werden diese in die Lücke zwischen Aufnahmenut 23 und Kugelkalotte 21 eingeklinkt, wobei das zweite Gelenkelement 2' vorübergehend leicht vom ersten Gelenkelement 2 abgehoben wird. Es ist auch möglich, dass diese provisorische Befestigung über eine Art Schnappverschluss erfolgt, der den Pin provisorisch an Ort und Stelle hält.

Gemäss einer weiteren, nicht in den Figuren dargestellten Ausführungsform, ist die Lage der Aufnahmenut in Bezug auf die beiden Gelenkelemente zur vorgängig beschriebenen Variante vertauscht. Dabei ist die Aufnahmenut an einer Kugelkalotte eines ersten Gelenkelements angeordnet und das benachbarte, zweite Gelenkelement weist eine Gelenkpfanne ohne Aufnahmenut auf. Kugelkalotte und Gelenkpfanne bilden wiederum zusammen ein Kugelgelenk und bei der Zugkraftbeaufschlagung des zentralen Zugkraftelements ist ein Pin zwischen Aufnahmenut und Gelenkpfanne form- und kraftschlüssig fixierbar.

Basierend auf der vorliegenden Offenbarung ist es offensichtlich für den Fachmann, dass die Geometrie der Aufnahmenut an die Aussenkontur der Pins angepasst ist. Soll eine zusätzliche Verdrehsicherung der Pins in der fixierten Stellung erreicht werden, so können entweder die zusammenwirkenden Oberflächen entsprechend aufgeraut, strukturiert oder zum Beispiel im Querschnitt mehrkantig ausgebildet werden.

Die Gelenkelemente 2 verfügen alle über einen Längsdurchgang 24, welcher unabhängig von der Formgebung der Stütze 11 der Vorrichtung 1 einen von der Spannungsvorrichtung am hinteren Ende bis zum Widerlager am vorderen Ende der Vorrichtung verlaufenden, durchgehenden Kanal bildet, in dem das Zugkraftelement 3 verläuft. Im gezeigten Beispiel besteht der Längsdurchgang 24 aus einem gegen die Kugelkalotte 21 offenen 241 zylindrischen Hohlraum 244, in dessen Boden eine kleinere untere Öffnung 242 zur Gelenkpfanne 22 führt. Grundsätzlich ist es bevorzugt, dass das Zugelement durch das Zentrum des Radius der Kugelkalotte geführt ist.

Der Längsdurchgang kann natürlich auch kegelförmig gestaltet werden, wobei jedoch eine zylinderförmige Ausgestaltung bei spanabhebenden Herstellungsverfahren vorteilhaft ist. Prinzipiell ist bei der Gestaltung der Längsdurchgangs zu beachten, dass die Auflagefläche zwischen Kugelkalotte und Gelenkpfanne des Kugelgelenks in allen Orientierungen des Kugelgelenks und auf alle Seiten hin ausreichend ist, um eine stabile Abstützung des Gelenks unter Zug sicherzustellen, und ein Abknicken der Stütze zu verhindern.

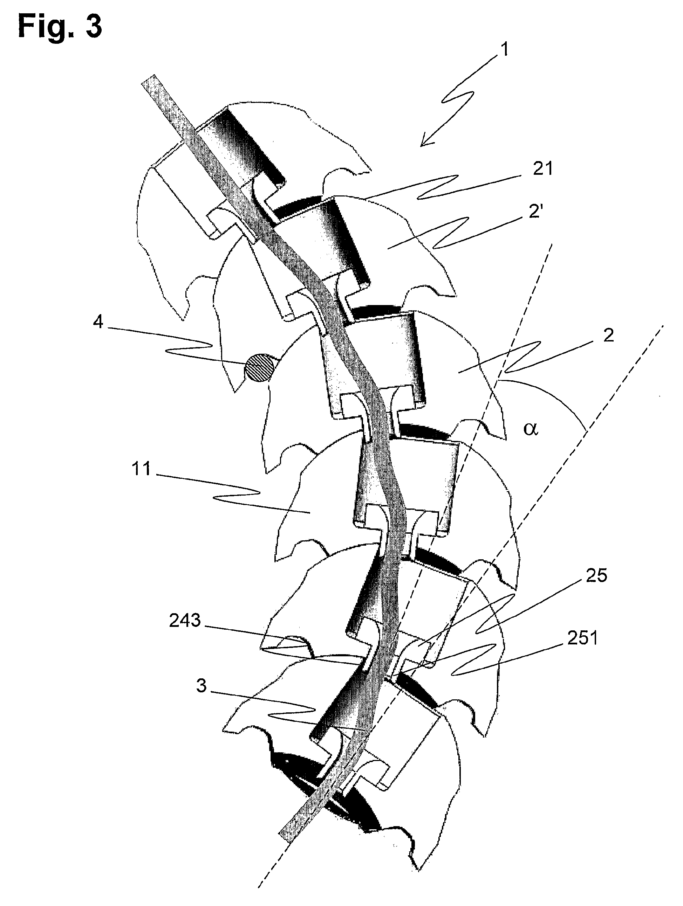

Figur 3 zeigt im Längsschnitt einen Abschnitt der Stütze 11 der gleichen Vorrichtung 1 wie in Figur 2, bei maximaler Biegung der Vorrichtung. Im Prinzip wird der maximale Verschwenkwinkel α zweier Gelenkelemente 2, 2' zueinander durch den Rand 243 der oberen Öffnung 241 des Längsdurchgangs 24 bestimmt, an dem bei maximaler Schwenkung das Zugkraftelement 3 anschlägt. Beim gezeigten Beispiel kann der Verschwenkwinkel bis zu 15° betragen. Das Anschlagen des Zugkraftelements 3 kann jedoch dazu führen, dass am Rand 243 auf das Zugkraftelement 3 Scherkräfte wirken, was zu Verschleisserscheinungen führen kann. Zudem sind die für das Zugkraftelement 3 besonders vorteilhaften Kohlefasermaterialien trotz extrem hoher Zugfestigkeit sehr empfindlich gegenüber solchen Scherkräften. Im gezeigten Ausführungsbeispiel des Gelenkelements 2 ist deshalb an der unteren Öffnung 242 des Längsdurchgangs 24 ein Umlenkelement 25 angeordnet, welches das Zugkraftelement 3 vor der Kante der unteren Öffnung 242 schützt, und den minimalen Biegeradius der zentralen Zugkraftelements 3 nach unten begrenzt. Das Umlenkelement 25 verfügt über einen Zapfen 251, welcher durch die untere Öffnung 242 hindurch verläuft, und über die Gelenkpfanne 22 hinaus ragt. Der Zapfen 251 bildet so einerseits einen Anschlag, der die maximale Verschwenkung der Gelenkelemente 2, 2' zueinander begrenzt, und andererseits schützt der genannte Zapfen 251 das Zugkraftelement vor der Kante 243 der oberen Öffnung 241 des anschliessenden Gelenkelements (2).

Um das Zugkraftelement 3 auch vor potentiell schädlichen Scherkräften aufgrund der Rotation der Gelenkelemente 2, 2' gegeneinander zu schützen, sollte das Umlenkelement 25 einen möglichst geringen Reibungskoeffizienten aufweisen, so dass das Zugkraftelement 3 und die Gelenkelemente 2, 2' in Bezug auf Rotation im Wesentlichen mechanisch entkoppelt sind. Das Material des Umlenkelementes ist so gewählt, dass das Zugelement nicht einschneidet und dass es sich nicht deformiert, wenn das Zugelement anliegt und angezogen wird. Gelenkelement 2 und Umlenkelement 25 können auch einstückig geformt sein, was insbesondere bei einer Herstellung im Spritzgussverfahren kosteneffizient machbar ist.

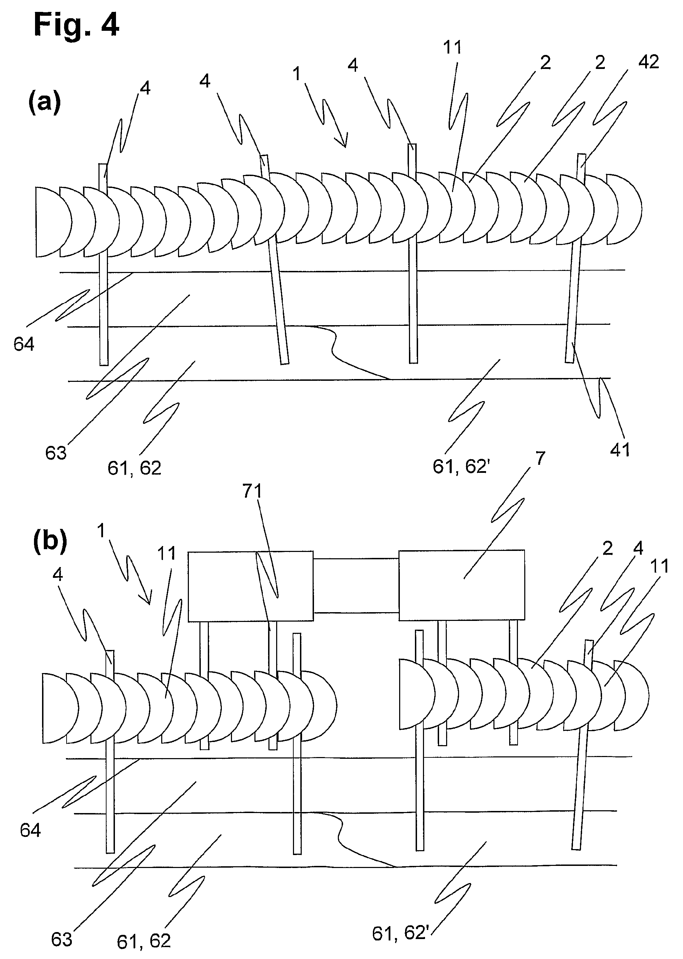

Die Anwendung einer erfindungsgemässen Vorrichtung wird in Figur 4 stark schematisiert dargestellt. Die Position der Pins im und relativ zu den einzelnen Knochenfragmenten ist in der Zeichnung nur grob angedeutet, beim tatsächlichen Einsatz gehen die Pins nicht nur durch eine Kortikalis, sondern sind bikortikal positioniert. Nach der Reposition der Fraktur werden in das Knochengewebe 61 der einzelnen Knochenfragmente 62, 62' eines Patienten mit den bekannten Methoden eine Mehrzahl von Pins 4 verankert. In der Regel werden vier bis sechs Pins gesetzt. Die Pins 4 können zur Anwendung mit der erfindungsgemässen Vorrichtung 1 ohne grosse Rücksichtnahme auf den noch anzubringenden Fixateur externe positioniert werden. Bei der Einstellung von offenen Frakturen in einer Notfallsituation, wo später noch eine definitive Osteosynthese mittels Platte oder Nagel erfolgt, wird in aller Regel nicht auf den Millimeter genau korrigiert; hier sind die Fragmente gelegentlich noch bis zu 5 bis 10 mm disloziert. Im Anbetracht der Begleitverletzungen ist eine schnelle Versorgung wichtig, und nicht die perfekte Reposition des Bruchs. Wichtig ist primär, dass keine Fragmente gegen die Haut, die Nerven, die Muskeln oder die Blutgefässe drücken. Bei definitiven Osteosynthesen hingegen wird versucht, bei der Reposition weniger als 5 mm Dislokation und 5° Achsabweichung zu erreichen.

Nach dem Setzen der Pins 4 und einer ersten, provisorischen Reposition der Fragmente werden die Pins 4 einfach an die erfindungsgemässe Fixateur-Vorrichtung angeklinkt. Durch die Verschwenkbarkeit von ca. 15° zwischen benachbarten Gelenkelementen und deren freie Drehbarkeit können auch schräg zueinander stehende Pins 4 problemlos fixiert werden. Auch die Abstände zwischen den Pins können im Wesentlichen ohne Abmessen gewählt werden. Die flexibel formbare Stütze 11 der erfindungsgemässen Vorrichtung kann diese Unregelmässigkeiten problemlos nachvollziehen.

Bei einer bevorzugten Version der erfindungsgemässen Vorrichtung ist bereits im Montagezustand das Zugkraftelement mit einer ersten, geringeren Zugkraft zwischen 100 und 200 N beaufschlagt, so dass zwischen den einzelnen Gelenkelementen 2 bereits ein Reibschluss vorhanden ist. Diese Vorspannung ist derart gewählt, das der Reibschluss beim manuellen Richten problemlos überwunden werden kann, und genügend Spiel vorhanden ist, dass die Pins in die Aufnahmenuten 23 der Gelenkelemente 2 eingeklinkt werden können, und der Kraft- und Reibschluss zwischen Pins und Gelenkelementen ein spontanes Verrutschen der Pins verhindert. Ein weiterer vorteilhafter Effekt dieser geringen Vorspannung besteht darin, dass die gliederkettenartige Stütze nicht so wackelig ist und beim Reponieren nicht durchhängt. Die Vorspannung ist nur so gross gewählt, dass die Gelenkelemente noch problemlos gegeneinander bewegt werden können, um die Fraktur zu reponieren. Die geringe Vorspannung ist entsprechend zu gering um die Fraktur reponiert zu halten.

Zum Erzeugen der Vorspannung kann, wie zum Beispiel in der Figur 1b dargestellt, ein Federelement 52 verwendet werden, das nicht nur die Kraft für die Vorspannung bereitstellt, sondern auch den Federweg zum einklinken der Pins.. Die erfindungsgemässe Vorrichtung ist im vorgespannten Zustand auch nach dem Einklinken der Pins 4 weiterhin von Hand beweglich, so dass der Bruch korrekt gerichtet werden kann. Dies kann mittels Bildwandlerdurchleuchtung kontrolliert werden. Wenn die Stellung der Knochenfragmente passt, wird die Spannungsvorrichtung (in Figur 4 nicht dargestellt) betätigt, wodurch mit dem Zugkraftelement die Gelenkelemente 2 gegeneinander gezogen und infolge Haftreibung blockiert werden. Die erfindungsgemässe Fixateur-Vorrichtung ist nun versteift. Die Position der Pins 4 wird bei diesem Vorgang des Versteifens nicht verändert. Um die Fraktur reponiert zu halten muss eine grössere Zugkraft von 500 bis 2000 N angelegt werden. Dies ist vorzugsweise durch manuelle Bedienung möglich. Die Vorrichtung ist nun provisorisch gesichert. Anschliessend wird vorzugsweise mit einer Aktuatorvorrichtung die Zugkraft auf einen definitiven Wert von 5000 bis 15000 N erhöht.

In weiteren, nicht in den Figuren dargestellten Ausführungsformen wird ein zweistufiger motorisierter Drehmomentschlüssel eingesetzt, der es erlaubt eine erste, leichtere Stufe einer Vorspannung aufzubauen. Eine zweite Spannungsstufe mit grösserer Vorspannung wird erst aufgebaut, wenn die Feder vollständig zusammengedrückt ist. Zur definitiven Fixierung des Fixateurs wird die zweite, hohe Stufe des zweistufigen Drehmomentschlüssels angeschaltet. Der Gegenhalt hierbei muss mit einem Werkzeug wie zum Beispiel einer Zange oder einem Gabelschlüssel gehalten werden. Alternativ kann diese definitive Fixierung mit einem manuellen Drehmomentschlüssel erfolgen.

Vorzugsweise ist die erfindungsgemässe Vorrichtung derart ausgelegt, dass die Spannung über mehrere Monate gar nicht oder nur unwesentlich nachlässt. Es ist auch möglich, eine erfindungsgemässe Vorrichtung mit einem optischen Indikator auszustatten, der beispielsweise je nach Zugkraft und/oder Modus (Montagemodus, Spannmodus erste und zweite Stufe, etc.) verschiedene Farben, Zahlen und/oder Buchstaben zeigt. Ebenfalls können Drucksensoren verschiedener Art zur Bestimmung und Überwachung der tatsächlich anliegenden Zugkraft verwendet werden.

Es kann notwendig sein, nachträglich weitere Pins zu setzten. In seinem solchen Fall muss die bereits versteifte Vorrichtung deblockiert werden, um die neuen Pins an die Stütze anzuklinken. Für das nachträgliche Anbringen werden jedoch bevorzugt Adapterelemente verwendet, die auf der versteiften erfindungsgemässen Vorrichtung reversibel form- und/oder kraftschlüssig angebracht werden können, und die über eine geeignete Haltevorrichtung für den zusätzlichen Pin verfügen. Beispielsweise zeigt WO 2007/001945 A1 eine dazu geeignete Klemmvorrichtung. Solche Adapterelemente können auch für die mechanische Kopplung mit anderen Fixateur-Vorrichtungen verwendet werden.

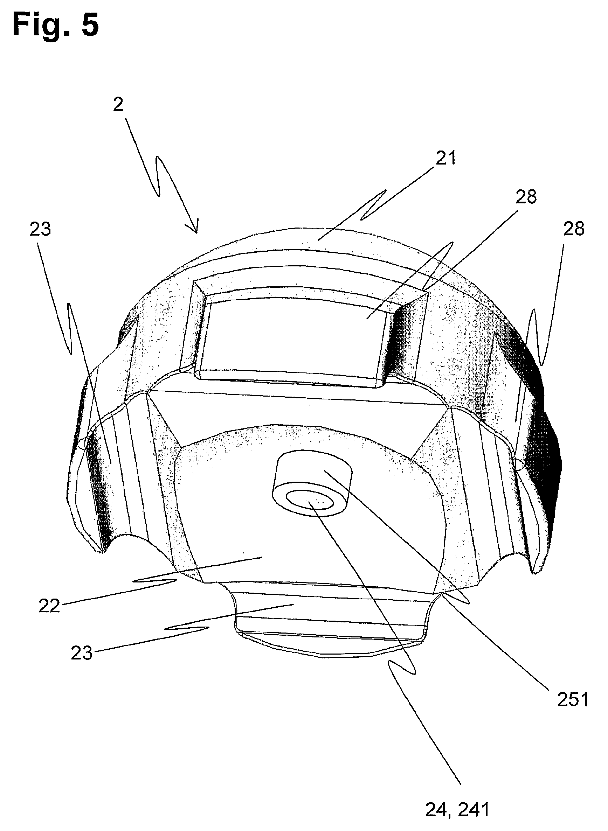

Ein einzelnes Gelenkelement 2 der erfindungsgemässen Vorrichtung aus Figur 1 ist in Figur 5 dargestellt, mit Blick halbschräg auf die Gelenkpfanne 22. Das Gelenkelement 2 hat im wesentlichen die äussere Form eines kurzen Zylinders, mit einer Kugelkalotte 21 an einem oberen Ende und einer Gelenkpfanne 22 am entgegengesetzten, unteren Ende, aus dem der Zapfen 251 des Umlenkelements 25 ragt. An der umlaufenden Zylinderfläche sind vier Zähne 28 angeformt, wobei zwischen einem Zahn 28 und der Gelenkpfanne 22 jeweils eine Nut 23 zur Aufnahme eines Pins vorhanden ist. Bei einer Zugspannung von 5000 N ist für diese Pin-Halterung ein Klemm-Drehmoment von 1 bis 5 Nm erreichbar. Grundsätzlich sollte der Abstand zwischen Aufnahmenut 23 und dem Zugkraftelement 3 möglichst gering sein, um eine möglichst gute Klemmwirkung zu erzielen. Die Oberfläche der Aufnahmenut 23 kann geeignet gestaltet werden, um einen möglichst hohen Reibungskoeffizienten zu erreichen, beispielsweise durch ein entsprechendes Profil, oder durch eine reibungserhöhende Beschichtung.

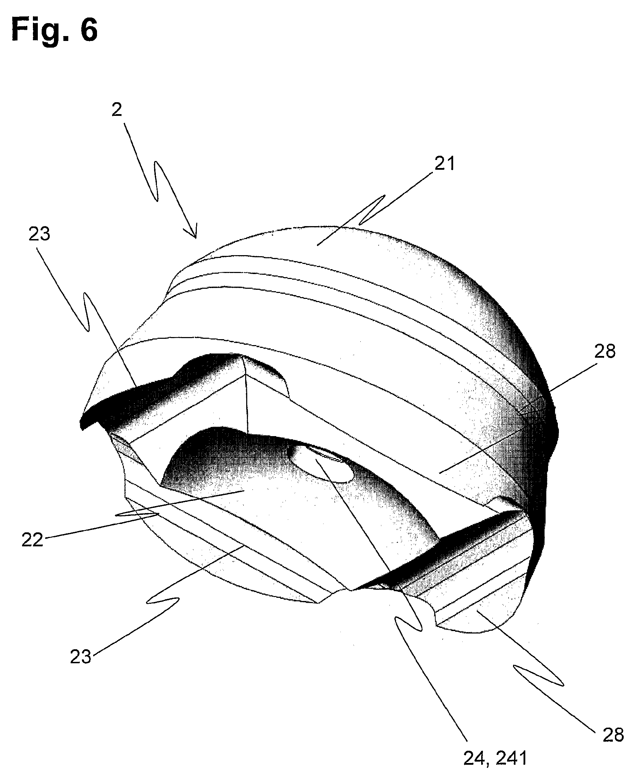

Figur 6 zeigt eine weitere mögliche Ausführungsform eines einzelnen Gelenkelements 2 einer erfindungsgemässen Vorrichtung, wie sie in den Figuren 2 und 3 dargestellt ist. Das Umlenkelement mit Zapfen ist weggelassen, um die Durchgangsöffnung 24, 241 sichtbar zu machen. Diese Variante ist besonders geeignet für eine spanabhebende Herstellungsweise. Nach dem Drehen eines rotationssymmetrischen Grundkörpers, und dem Bohren des Längsdurchgangs 24 werden die vier Aufnahmenuten 23 ausgefräst.

Der Durchmesser eines Gelenkelements einer erfindungsgemässen Vorrichtung beträgt typischerweise zwischen 30 und 40 mm, wobei hier ein Kompromiss zwischen mechanischer Stabilität und Platzverbrauch gewählt werden muss. So beträgt beispielsweise bei der Variante der erfindungsgemässen Vorrichtung aus Figur 1 der Durchmesser der Gelenkelemente 2 bevorzugt 40 mm, die Höhe 23 mm, und der Kugelradius der Kugelkalotte 20 mm. In gestreckten Zustand resultiert so ein Abstand von 20 mm zwischen zwei einzelnen Gelenkelementen. Die Formgebung und Position der Aufnahmenuten 23 ist auf den Durchmesser der zu klemmenden Pins angepasst, welcher standardmässig 5 mm beträgt, aber auch die ebenfalls gebräuchlichen Pins mit einem Durchmesser von 4 mm oder 6 mm aufnehmen kann. Machbar sind auch Gelenkelemente mit verschiedenen Nuten für verschiedene Pin-Durchmesser. Im Prinzip wäre nur eine Aufnahmenut notwendig, da die Gelenkelemente einer erfindungsgemässen Vorrichtung frei um ihre Längsachse drehbar sind.

Bei einer weiteren möglichen Ausgestaltungsform einer erfindungsgemässen Vorrichtung kann auch auf der Kugelkalotte 21 eine Nut angebracht sein, so dass ein Pin sowohl in der Aufnahmenut 23 als auch in der Nut auf der Kugelkalotte 21 liegt. Alternativ kann die Nut auch als die Kugelkalotte 21 umlaufende Ringnut ausgestaltet werden. Diese Variante bietet den Vorteil der grösseren Kontaktfläche zwischen Pin und Kugelkalotte, führt jedoch auch dazu, dass die zwei beteiligten Gelenkelemente entlang ihrer Längsachse gefluchtet und mit korrektem Rotationswinkel ausgerichtet sein müssen, was die Flexibilität der Stütze an dieser Stelle reduziert.

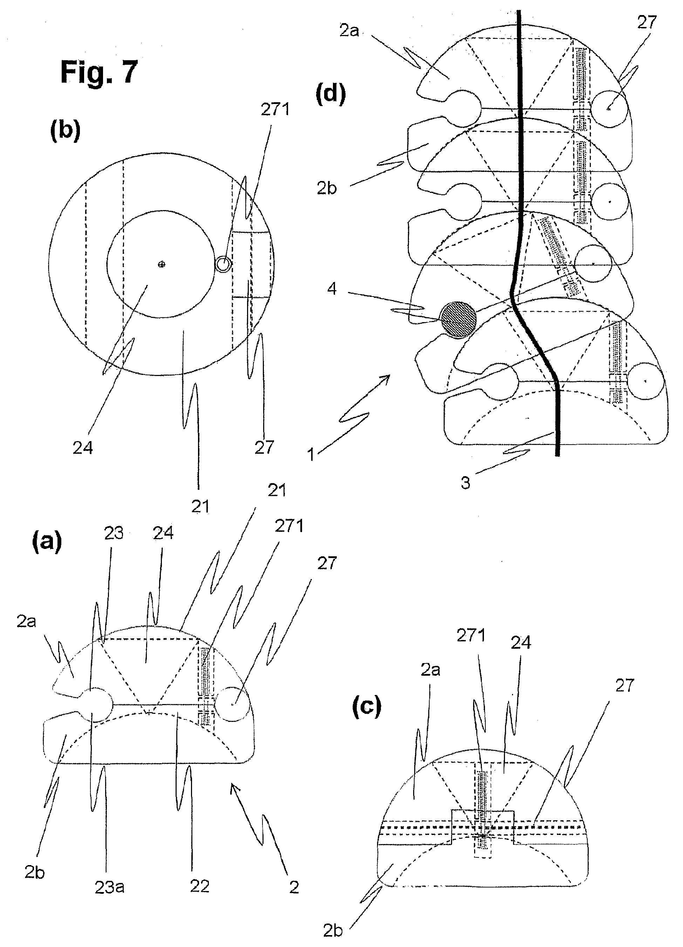

Anstatt als einteiliger Körper können die Gelenkelemente auch zweiteilig ausgeführt sein. So zeigt Figur 7 beispielsweise eine zweiteilige Ausführungsform eines Gelenkelements 2, mit einem oberen Teil 2a mit einer Kugelkalotte 21 und einem mit diesem über ein Scharniergelenk 27 schwenkbeweglich verbundenen unteren Teil 2b mit einer Gelenkpfanne 22, (a) in Seitenansicht mit Blick auf die Scharnierachse, (b) in Aufsicht mit Blick entlang der Längsachse, (c) in Seitenansicht mit Blick senkrecht zur Scharnierachse, und (d) in einem Abschnitt einer Stütze 11 entsprechenden erfindungsgemässen Vorrichtung 1. Auf den einander gegenüberliegenden inneren Seiten der beiden Teile 2a, 2b sind zwei parallele Aufnahmenuten 23, 23a angeordnet, zwischen denen ein Pin festgeklemmt werden kann. Da die Zugkraft des Zugkraftelements 3 im Wesentlichen senkrecht zur Achse des Scharniergelenks 27 verläuft, kann das Gelenk 27 vergleichsweise leicht gebaut sein. Die genannte Ausführungsform ist besonders robust, und erlaubt eine grössere Nähe der Aufnahmenuten 23, 23a zum Zugkraftelement 3, und damit ein höheres Klemm-Drehmoment.

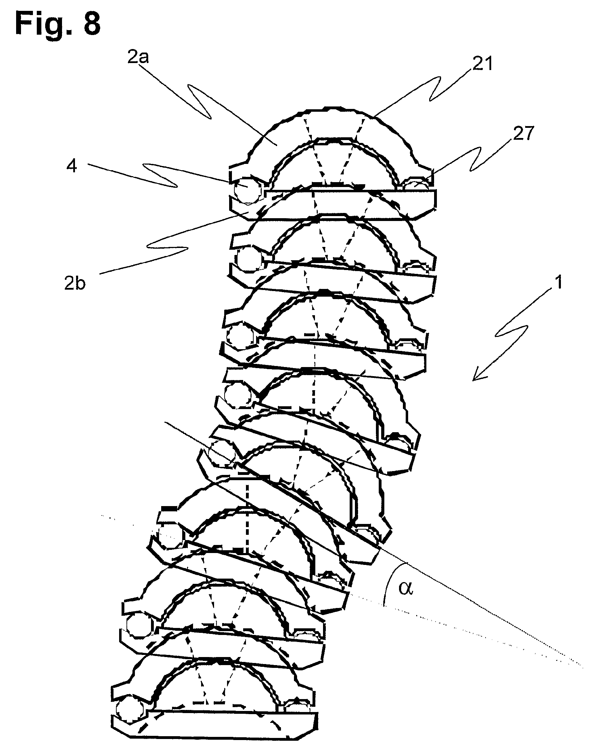

Wie auch bei den oben stehend besprochenen einteiligen Ausführungsformen der Gelenkelemente wird auch bei der zweiteiligen Variante die Klemmkraft zum Fixieren des Pins durch das zentrale Zugkraftelement 3 erzeugt. Das Beispiel aus Figur 7 verfügt zusätzlich noch über ein weiteres Federelement 271, beispielsweise in Form einer Zugfeder, welches die beiden Teile 2a, 2b zusätzlich mit einer geringeren Federkraft beaufschlagt, so dass bei der Montage der erfindungsgemässen Vorrichtung 1 die Pins provisorisch befestigt werden können. Diese integrierten Federelemente 271 können auch weggelassen werden, wobei dann eine Federkraft zum provisorischen Befestigen analog zur einteiligen Ausführungsform erfolgt. Eine weitere Variante einer erfindungsgemässen Vorrichtung 1 mit zweiteiligen 2a, 2b Gelenkelementen 2 und ohne Federelement ist in Figur 8 dargestellt.

In einer weiteren bevorzugten Ausführungsform weist eine erfindungsgemässe Vorrichtung Kopplungsmittel auf, um die Vorrichtung mit anderen Fixateur-Vorrichtungen oder Zusatzvorrichtungen zu koppeln. Beispielsweise kann an einem vorderen Ende der erfindungsgemässen Vorrichtung eine Schraubkopplung angebracht sein, mit der diese mit einem vorderen Ende einer anderen erfindungsgemässen Vorrichtung oder einem Kopplungselement verbindbar ist. Ebenfalls denkbar ist ein Kopplungselement beispielsweise in der Form einer Platte mit mehreren geeignet angeordneten Pins, welche in zwei zu verbindende erfindungsgemässe Vorrichtungen eingeklinkt werden können, und diese so verbinden. Auf diese Art und Weise können auch Zwischensegmente eingebracht werden, beispielsweise zur Überbrückung grösserer Distanzen. Längere Fixateur externe, beispielsweise von der Hüfte bis zum Sprunggelenk, werden vorzugsweise aus zwei oder mehr einzelnen erfindungsgemässen Vorrichtungen zusammengesetzt, da die notwendigen Zugkräfte proportional zur Länge der Fixateur-Vorrichtung wachsen. Bei Bedarf können weitere Bestandteile in die erfindungsgemässe Fixateur-Vorrichtung integriert werden, die beispielsweise eine Distraktion, Achskorrektur oder Verlängerung zulassen, und über die so Einfluss auf die Frakturheilung genommen werden kann. Beispiele sind Distraktionselemente und dämpfende Federelemente. Es ist auch möglich, einzelne oder alle Gelenkelemente verlängerbar auszuführen.

Eine erfindungsgemässe Vorrichtung kann auch über ein geeignetes Kopplungselement sternförmig mit zwei oder mehr zusätzlichen erfindungsgemässen Vorrichtungen verbunden werden. Auf diese Weise ist beispielsweise ein Hybridfixateur realisierbar, der eine erfindungsgemässe Vorrichtung aufweist, die an Pins befestigt wird, und die an einem Kopplungselement in zwei bis vier Arme übergeht, die ebenfalls als erfindungsgemässe Vorrichtung ausgestaltet sind, und die mit einem Ringfixateur verbunden sind.

Bei einem erfindungsgemässen Verfahren können auch Gruppen von Pins auf zwei Seiten einer Fraktur mit je einer erfindungsgemässen Vorrichtung respektive einer Stütze 11 verbunden werden. Zwischen diesen wird dann anschliessend ein Zwischenelement 7 eingebaut, welches beispielsweise eine nachträgliche Distraktion oder Achskorrektur zulässt, zum Beispiel bei einer Nachkorrektur auf der Station oder in der Sprechstunde. Solch eine Ausführungsform mit einem Zwischenelement ist in Figur 4(b) schematisch dargestellt. Das Zwischenelement 7 ist über vier Pins 71 mit den beiden Stützen 11 der Vorrichtungen 1 verbunden. In einem solchen Zwischenelement 7 können auch Dämpferelemente integriert werden, die eine Einstellung der Härte der Gesamtkonstruktion zulassen. Eine solche elastische Fixierung von Frakturen kann etwa gewünscht sein, um in bestimmten Fällen einen besseren Heilungsverlauf zu erreichen.

Gemäss weiterer, nicht in den Figuren dargestellter Ausführungsformen der Erfindung, sind zwei oder mehr kurze Stützelemente oder Teilelemente zu einer Stütze mit einer gewünschten Länge verbunden. Die einzelnen Teilelemente werden zusammen geklinkt um einerseits die definitive Länge der benötigten Stütze zu erreichen, andererseits um den benötigten Typ des Fixateurs bereitzustellen. So kann ein Fixateur im Sinne einer einzigen Schlange (für den mehrfachverletzten Traumapatienten in der Nacht), oder aber ein Fixateur mit einem Schlangenelement an beiden Enden und einem Dämpferelement oder einem Korrekturelement in der Mitte (für die definitive Ausbehandlung einer Fraktur) zusammen gesetzt werden. Die Elemente werden durch Kupplungen verbunden, diese Kupplungen ermöglichen einerseits die Verbindung der Elemente, andererseits die Übertragung des zentralen Zugs (von Zugelement von Element 1 auf das Zugelement von Element 2 auf das Zugelement von Element 3). Das heisst, es ist trotz Kupplung mehrerer Elemente weiterhin nur eine Spannvorrichtung an einer Seite nötig.

Gemäss weiterer vorteilhafter Ausführungsformen können an den erfindungsgemässen Vorrichtungen auch beidseitig Spannvorrichtungen angeordnet sein.

Gemäss weiterer bevorzugter Ausführungsformen sind die Kugelgelenkelemente aus einem geeigneten Kunststoff oder Metall gefertigt.

Gemäss einem vorteilhaften Verfahren zur externen Fixierung von gebrochenen Knochen 62, 62' eines Patienten, insbesondere der Extremitäten, werden eine Mehrzahl von Pins 4 mit ihrem proximalen Ende 41 im Knochengewebe 61 des Patienten verankert und die distalen Enden 42 der Pins 4 werden provisorisch an einer Stütze 11 einer oben beschriebenen erfindungsgemässen Vorrichtung 1 befestigt. Anschliessend werden die Fragmente 62, 62' in ihre im wesentlichen anatomische Ursprungslage repositioniert, und ein zentrales Zugkraftelement 3 der Vorrichtung 1 wird mit einer Zugkraft beaufschlagt, so dass die Stütze 11 der Vorrichtung 1 versteift und die Pins 4 an der Stütze 11 klemmend und/oder reibschlüssig fixiert. Vorzugsweise wird die auf das Zugkraftelement 3 wirkende Zugkraft gelöst, um die Reposition der Knochenfragmente korrigieren zu können. Anschliessend wird das Zugkraftelement 3 der Vorrichtung 1 erneut mit einer Zugkraft beaufschlagt, so dass die Stütze 11 der Vorrichtung wieder versteift wird und die Pins 4 an der Stütze 11 klemmend und/oder reibschlüssig fixiert werden.

### Bezugszeichenliste

- 1: Fixateur externe, Vorrichtung zur externen Fixierung
- 11: Stütze
- 2,2': Gelenkelement
- 2a, 2b: Teil eines Gelenkelements
- 21: Kugelkalotte
- 22, 22': Gelenkpfanne
- 23, 23a: Nut zur Aufnahme des Pins
- 24: Längsdurchgang
- 241: obere Öffnung
- 242: untere Öffnung
- 243: Rand
- 244: Hohlraum
- 25: Umlenkelement
- 251: Zapfen
- 27: Scharniergelenk
- 271: Federelement
- 28: Zahn
- 3: zentrales Zugkraftelement
- 4: Pin
- 41: proximales Ende
- 42: distales Ende
- 5: Spannungsvorrichtung
- 51: Bolzen
- 52: Federelement
- 53: Gehäuse
- 54: Platte
- 55: Widerlager
- 61: Knochengewebe
- 62, 62': Knochenfragment
- 63: Weichteilgewebe
- 64: Haut
- 7: Zwischenelement
- 71: Pin

## Patentansprüche

1. Vorrichtung (1) zur externen Fixierung von Knochenfragmenten (62, 62') eines Patienten, insbesondere der Extremitäten, mit einer außerhalb des Körpers des Patienten anordenbaren Stütze (11) aus einer Mehrzahl von aneinander gereihten Gelenkelementen (2, 2'), an der mindestens zwei mit ihrem proximalen Ende (41) im Knochengewebe (61) des Patienten verankerte, perkutan angeordnete Pins (4) mit ihrem distalen Ende (42) befestigbar und in ihrer räumlichen Lage zueinander fixierbar sind; wobei die Gelenkelemente (2) auf einem zentralen Zugkraftelement (3) aufgereiht sind, und jeweils zwei benachbarte Gelenkelemente (2, 2') zusammen ein Kugelgelenk mit Kugelkalotte (21) und Gelenkpfanne (22) bilden; und wobei die einzelnen Kugelgelenke und damit die Stütze (11) durch eine Zugkraftbeaufschlagung des zentralen Zugkraftelements (3) reversibel kraftschlüssig, vorzugsweise reibschlüssig fixierbar sind, **dadurch gekennzeichnet, dass** die Gelenkelemente (2, 2') derart ausgestaltet sind, dass ein Pin (4) durch die Zugkraftbeaufschlagung des zentralen Zugkraftelements (3) kraftschlüssig, vorzugsweise klemmend und/oder reibschlüssig, an der Stütze (11) befestigbar ist.

2. Vorrichtung nach Anspruch 1, **dadurch gekennzeichnet, dass** ein erstes Gelenkelement (2) der Stütze (11) eine Kugelkalotte (21), und ein benachbartes, zweites Gelenkelement (2') eine Gelenkpfanne (22) und eine Aufnahmenut (23) aufweist, wobei Kugelkalotte (21) und Gelenkpfanne (22) zusammen ein Kugelgelenk bilden, und wobei bei der Zugkraftbeaufschlagung des zentralen Zugkraftelements (3) ein Pin (4) zwischen Aufnahmenut (23) und Kugelkalotte (21) form- und kraftschlüssig fixierbar ist oder dass ein erstes Gelenkelement der Stütze eine Kugelkalotte und eine Aufnahmenut und ein benachbartes, zweites Gelenkelement eine Gelenkpfanne aufweist, wobei Kugelkalotte und Gelenkpfanne zusammen ein Kugelgelenk bilden, und wobei bei der Zugkraftbeaufschlagung des zentralen Zugkraftelements (3) ein Pin (4) zwischen Aufnahmenut und Gelenkpfanne form- und kraftschlüssig fixierbar ist.

3. Vorrichtung nach Anspruch 1, **gekennzeichnet durch** ein aus zwei schwenkbeweglich verbundenen Teilen (2a, 2b) bestehendes Gelenkelement (2), wobei ein erster Teil (2a) eine mit einer Gelenkpfanne (22) eines ersten benachbarten Gelenkelements (2) zusammenwirkende Kugelkalotte (21) aufweist, und ein zweiter Teil (2b) eine mit einer Kugelkalotte (21) eines zweiten benachbarten Gelenkelements (2) zusammenwirkende Gelenkpfanne (22) aufweist; und wobei bei der Zugkraftbeaufschlagung des zentralen Zugkraftelements (3) ein Pin (4) zwischen den beiden Teilen (2a, 2b) form- und kraftschlüssig fixierbar ist.

4. Vorrichtung nach einem der vorangehenden Ansprüche, **dadurch gekennzeichnet, dass** in einem Längsdurchgang (24) eines einzelnen Gelenkelements (2, 2'), durch den das zentrale Zugkraftelement (3) verläuft, mindestens ein Umlenkelement (25) angeordnet ist, welches den maximalen Biegewinkel des Zugkraftelements (3) begrenzt.

5. Vorrichtung nach einem der vorangehenden Ansprüche, **dadurch gekennzeichnet, dass** in einer Gelenkpfanne (22) eines ersten Gelenkelements (2) ein Zapfen (251) angeordnet ist, welcher die Schwenkbeweglichkeit eines durch die Gelenkpfanne (22) und eine Kugelkalotte (21) eines benachbarten, zweiten Gelenkelements (2) gebildeten Kugelgelenks beschränkt, indem er in eine auf der Kugelkalotte (21) gelegene obere Öffnung (241) des Längsdurchgangs (24) des zweiten Gelenkelements (2) hineinragt.

6. Vorrichtung nach einem der vorangehenden Ansprüche, **dadurch gekennzeichnet, dass** die Gelenkelemente (2, 2') aus Metall, einem faserverstärkten Polymermaterial oder einem geeigneten Kunststoffmaterial bestehen.

7. Vorrichtung nach einem der vorangehenden Ansprüche, **dadurch gekennzeichnet, dass** das zentrale Zugkraftelement (3) ein Bündel ist, insbesondere ein Drahtbündel, ein Kohlefaserbündel oder ein Bündel aus einem anderen geeigneten Kunststoffmaterial oder einer Kombination der vorgenannten Materialien.

8. Vorrichtung nach einem der vorangehenden Ansprüche, **gekennzeichnet durch** ein mit dem zentralen Zugkraftelement (3) verbundenes Federelement (52), mittels dem das zentrale Zugkraftelement (3) mit einer Zugkraft beaufschlagbar ist.

9. Vorrichtung nach einem der vorangehenden Ansprüche, **gekennzeichnet durch** eine Spannungsvorrichtung (5), mittels derer eine auf das zentrale Zugkraftelement (3) wirkende Zugkraft erzeugbar ist.

10. Vorrichtung nach Anspruch 9, **dadurch gekennzeichnet, dass** die Spannungsvorrichtung (5) an einem Ende der Stütze (11) der Vorrichtung (1) angeordnet und/oder demontierbar ist.

## Claims

1. A device (1) for the external fixation of bone fragments (62, 62') of a patient, in particular of the extremities, having a support (11) which can be disposed outside the body of the patient, formed from a plurality of joint elements (2, 2') which are strung together, to which at least two percutaneously disposed pins (4) anchored with their proximal end (41) in the bone tissue (61) of the patient can be secured via their distal end (42) and can be fixed in their spatial position relative to each other; wherein the joint elements (2) are strung together on a central tensile force element (3), and respectively two adjacent joint elements (2, 2') together form a ball joint with a spherical cap (21) and a joint socket (22); and wherein the individual ball joints, and therefore the support (11), can be reversibly fastened by means of a force fit, preferably a frictional fit, by applying a tensile force to the central tensile force element (3), **characterized in that** the joint elements (2, 2') are configured in a manner such that by applying the tensile force via the central tensile force element (3), a pin (4) can be fastened to the support (11) by means of a force fit, preferably by clamping and/or by a frictional fit.

2. The device according to claim 1, **characterized in that** a first joint element (2) of the support (11) has a spherical cap (21), and an adjacent, second joint element (2') has a joint socket (22) and a receiving groove (23), wherein the spherical cap (21) and joint socket (22) together form a ball joint, and wherein a pin (4) can be fastened between the receiving groove (23) and the spherical cap (21) by form fitting and force fitting when applying the tensile force to the central tensile force element (3), or **in that** a first joint element of the support has a spherical cap and a receiving groove, and an adjacent second joint element has a joint socket, wherein the spherical cap and joint socket together form a ball joint, and wherein a pin (4) can be fastened between the receiving groove and the joint socket by form fitting and force fitting when applying the tensile force to the central tensile force element (3).

3. The device according to claim 1, **characterized by** a joint element (2) consisting of two pivotably connected parts (2a, 2b), wherein a first part (2a) has a spherical cap (21) which cooperates with a joint socket (22) of a first adjacent joint element (2), and wherein a second part (2b) has a joint socket (22) which cooperates with a spherical cap (21) of a second adjacent joint element (2); and wherein a pin (4) can be fastened between the two parts (2a, 2b) by form fitting and force fitting when applying the tensile force to the central tensile force element (3).

4. The device according to any one of the preceding claims, **characterized in that** at least one deflection element (25) which limits the maximum bending angle of the tensile force element (3) is disposed in a longitudinal passage (24) of an individual joint element (2, 2'), through which the central tensile force element (3) extends.

5. The device according to any one of the preceding claims, **characterized in that** a stud (251) is disposed in a joint socket (22) of a first joint element (2) which limits the pivotability of a ball joint formed by the joint socket (22) and by a spherical cap (21) of an adjacent second joint element (2) by protruding into an upper opening (241) of the longitudinal passage (24) of the second joint element (2) located on the spherical cap (21).

6. The device according to any one of the preceding claims, **characterized in that** the joint elements (2, 2') are produced from metal, a fibre-reinforced polymeric material or a suitable synthetic material.

7. The device according to any one of the preceding claims, **characterized in that** the central tensile force element (3) is a bundle, in particular a wire bundle, a carbon fibre bundle or a bundle of another suitable synthetic material, or a combination of said materials.

8. The device according to any one of the preceding claims, **characterized by** a spring element (52) which is connected to the central tensile force element (3), by means of which a tensile force can be applied to the central tensile force element (3).

9. The device according to any one of the preceding claims, **characterized by** a tensioning device (5), by means of which a tensile force acting on the central tensile force element (3) can be generated.

10. The device according to claim 9, **characterized in that** the tensioning device (5) is disposed on one end of the support (11) of the device (1) and/or the tensioning device (5) is detachable.

## Revendications

1. Dispositif (1) de fixation externe de fragments d'os (62, 62') d'un patient, en particulier des extrémités, avec un soutien (11) disposable en dehors du corps du patient composé d'une pluralité d'éléments articulaires (2, 2') alignés l'un contre l'autre, sur lesquels au moins deux broches (4) disposées de manière percutanée, ancrées avec leur extrémité proximale (41) dans le tissu osseux (61) du patient peuvent être fixées avec leur extrémité distale (42) et peuvent être fixées dans leur position spatiale l'une par rapport à l'autre, les éléments articulaires (2) étant alignés sur un élément de force de traction central (3) et deux éléments articulaires (2, 2') voisins formant respectivement ensemble une articulation orbiculaire avec calotte sphérique (21) et cavité glénoïde (22) et les articulations orbiculaires individuelles et de ce fait le soutien (11) pouvant être fixés par une application de force de traction de l'élément de force de traction central (3) de façon réversible par conformité de force, de préférence par conformité de friction, **caractérisé en ce que** les éléments articulaires (2, 2') sont configurés de telle manière qu'une broche (4) peut être fixée au soutien (11) par l'application d'une force de traction de l'élément de force de traction central (3) par conformité de force, de préférence de manière bloquante et/ou par conformité de friction.

2. Dispositif selon la revendication 1, **caractérisé en ce qu'**un premier élément articulaire (2) du soutien (11) comporte une calotte sphérique (21) et un deuxième élément articulaire (2') voisin comporte une cavité glénoïde (22) et une rainure de réception (23), la calotte sphérique (21) et la cavité glénoïde (22) formant ensemble une articulation orbiculaire et une broche (4) pouvant être fixée par conformité de forme et de force entre la rainure de réception (23) et la calotte sphérique (21) lors de l'application de la force de traction de l'élément de force de traction central (3) ou en ce qu'un premier élément articulaire du soutien comporte une calotte sphérique et une rainure de réception et un deuxième élément articulaire voisin comporte une cavité glénoïde, la calotte sphérique et la cavité glénoïde formant ensemble une articulation orbiculaire et une broche (4) pouvant être fixée par conformité de forme et de force entre la rainure de réception et la cavité glénoïde lors de l'application de la force de traction de l'élément de force de traction central (3).

3. Dispositif selon la revendication 1, **caractérisé par** un élément articulaire (2) composé de deux parties (2a, 2b) reliées de manière mobile en pivotement, une première partie (2a) comportant une calotte sphérique (21) coopérant avec une cavité glénoïde (22) d'un premier élément articulaire voisin (2) et une deuxième partie (2b) comportant une cavité glénoïde (22) coopérant avec une calotte sphérique (21) d'un deuxième élément articulaire voisin (2) et une broche (4) pouvant être fixée par conformité de forme et de force entre les deux parties (2a, 2b) lors de l'application de la force de traction de l'élément de force de traction central (3).

4. Dispositif selon l'une quelconque des revendications précédentes, **caractérisé en ce qu'**au moins un élément de déviation (25), limitant l'angle de flexion maximal de l'élément de force de traction (3), est disposé dans un passage longitudinal (24) d'un élément articulaire individuel (2, 2'), à travers lequel passe l'élément de force de traction central (3).

5. Dispositif selon l'une quelconque des revendications précédentes, **caractérisé en ce qu'**un pivot (251) est disposé dans une cavité glénoïde (22) d'un premier élément articulaire (2), lequel limite la pivotabilité d'une articulation orbiculaire formée par la cavité glénoïde (22) et une calotte sphérique (21) d'un deuxième élément articulaire (2) voisin, en se prolongeant dans une ouverture (241) supérieure située sur la calotte sphérique (21) du passage longitudinal (24) du deuxième élément articulaire (2).

6. Dispositif selon l'une quelconque des revendications précédentes, **caractérisé en ce que** les éléments articulaires (2, 2') sont en métal, en matériau polymère renforcé aux fibres ou dans une matière plastique appropriée.

7. Dispositif selon l'une quelconque des revendications précédentes, **caractérisé en ce que** l'élément de force de traction central (3) est un faisceau, en particulier un faisceau de fils, un faisceau de fibres de carbone ou un faisceau composé d'une autre matière plastique appropriée ou d'une combinaison des matériaux précités.

8. Dispositif selon l'une quelconque des revendications précédentes, **caractérisé par** un élément à ressort (52) relié à l'élément de force de traction central (3), au moyen duquel l'élément de force de traction central (3) est sollicité par une force de traction.

9. Dispositif selon l'une quelconque des revendications précédentes, **caractérisé par** un dispositif de tension (5) au moyen duquel une force de traction agissant sur l'élément de force de traction central (3) peut être générée.

10. Dispositif selon la revendication 9, **caractérisé en ce que** le dispositif de tension (5) est disposé à une extrémité du soutien (11) du dispositif (1) et/ou le dispositif de tension (5) est démontable.
